# EUROPÄISCHE PATENTANMELDUNG

(11) **EP 3 369 854 A1**
(43) Veröffentlichungstag der Anmeldung: **05.09.2018**
(21) Anmeldenummer: 18156972.4
(22) Anmeldetag: 15.02.2018
(51) Int. Cl.: D06F 39/00, D06H 3/08, G01N 33/36, G01N 21/88

(54) **SYSTEM**

(30) Priorität: 03.03.2017 DE 102017104483
(71) Anmelder: Miele & Cie. KG, 33332 Gütersloh (DE)
(72) Erfinder: Bicker, Manuela, 33415 Verl (DE); Bicker, Rainer, 33415 Verl (DE); Hügelmeyer, Christian, 33428 Harsewinkel (DE)

(57) **Zusammenfassung**

Die Erfindung betrifft ein System, welches ein elektrisches Gerät (1, 2) mit einer Aufnahmeeinheit (11, 21) zum Aufnehmen einer Digitalaufnahme und einer Ausgabeeinheit (12, 22) und eine Auswerteinheit zum Auswerten der Digitalaufnahme aufweist und welches ausgebildet ist, mittels der Aufnahmeeinheit (11, 21) eine Digitalaufnahme von zumindest einem Teil eines Wäschestücks aufzunehmen, mittels der Auswerteinheit anhand der Digitalaufnahme einen Fleck auf dem Wäschestück derart auszuwerten, dass der Fleck mindestens einem Standardfleck aus mehreren in dem System hinterlegten Standardflecken zugeordnet wird, welchen jeweils eine Behandlungsmethode zum Entfernen des Flecks zugeordnet ist, und mittels der Ausgabeeinheit (12, 22) die dem hinterlegten Fleck zugeordnete Behandlungsmethode zum Entfernen des Flecks auszugeben.

## Beschreibung

Die Erfindung betrifft ein System und insbesondere ein System zur optischen Fleckenerkennung auf einem Wäschestück. Manche Flecken sind auf Wäschestücken wie Textilien schwer zu identifizieren. Eine falsche Behandlungsmethode kann jedoch zu einer dauerhaften Fixierung des Flecks auf dem Wäschestück führen. Zudem kann auch bei bekannter Fleckenursache eine Behandlungsmethode unbekannt sein.

Der Erfindung stellt sich somit das Problem, ein System bereitzustellen, welches geeignet ist, einem Nutzer mit fleckigem Wäschestück eine geeignete Behandlungsmethode zum Entfernen eines oder mehrerer Flecken von dem Wäschestück vorzuschlagen.

Erfindungsgemäß wird dieses Problem durch ein System mit den Merkmalen des Patentanspruchs 1 gelöst. Vorteilhafte Ausgestaltungen und Weiterbildungen der Erfindung ergeben sich aus den nachfolgenden Unteransprüchen.

Die mit der Erfindung erreichbaren Vorteile bestehen neben dem Vorschlagen einer geeigneten Behandlungsmethode darin, dass ein Nutzer mit fleckiger Wäsche nicht selbst eine Behandlungsmethode heraus suchen und/oder finden muss, was ihm Zeit spart.

Die Erfindung betrifft ein System, welches ein elektrisches Gerät mit einer Aufnahmeeinheit zum Aufnehmen einer Digitalaufnahme und einer Ausgabeeinheit und eine Auswerteinheit zum Auswerten der Digitalaufnahme aufweist und welches ausgebildet ist, mittels der Aufnahmeeinheit eine Digitalaufnahme von zumindest einem Teil eines Wäschestücks aufzunehmen, mittels der Auswerteinheit anhand der Digitalaufnahme einen Fleck auf dem Wäschestück derart auszuwerten, dass der Fleck mindestens einem Standardfleck aus mehreren in dem System hinterlegten Standardflecken zugeordnet wird, welchen jeweils eine Behandlungsmethode zum Entfernen des Flecks zugeordnet ist, und mittels der Ausgabeeinheit die dem hinterlegten Fleck zugeordnete Behandlungsmethode zum Entfernen des Flecks auszugeben.

Das System ist geeignet, dem Nutzer des elektrischen Geräts auf einfache und zeit- und kostengünstige Weise eine Behandlungsmethode zum Entfernen eines oder mehrerer Flecken von dem Wäschestücken vorzuschlagen.

In dem System sind eine Mehrzahl an Behandlungsmethoden und eine Mehrzahl an Standardflecken hinterlegt, wobei jedem Standardfleck eine Behandlungsmethode zugeordnet ist. Der mit der Aufnahmeeinheit aufgenommene Fleck wird mittels der Auswerteinheit einem oder ggf. mehreren Standardflecken zugeordnet. Dazu wird der aufgenommene Fleck bevorzugt optisch mittels der Auswerteinheit ausgewertet. Beispielsweise wird die Fleckfarbe auf weißem Untergrund ermittelt. In dem System sind die Standardflecken vorzugsweise für verschiedene Fleckfarben hinterlegt, so dass sie mit dem aufgenommenen Fleck verglichen werden können. Ein oder mehrere dem aufgenommen Fleck entsprechende oder ähnliche Standardflecken können dem Fleck zugeordnet werden, so dass die ihm oder ihnen zugeordnete(n) Behandlungsmethode(n) ausgegeben werden können. Mit der Formulierung "dem aufgenommen Fleck entsprechende oder ähnliche Standardflecken" ist gemeint, dass die Fleckfarbe des Standardflecks der Fleckfarbe des aufgenommen entspricht oder zumindest ähnlich ist.

Die Aufnahmeeinheit ist bevorzugt eine Digitalkamera. Die Ausgabeeinheit kann ein Display und/oder einen Lautsprecher aufweisen, welche ausgebildet sind, dem Nutzer die Behandlungsmethode optisch und/oder auditiv mitzuteilen. Bevorzugt ist die Ausgabeeinheit als ein Display ausgebildet, das dem Nutzer des elektrischen Geräts die Behandlungsmethode anzeigt. Beispielsweise ist das Display ein Bildschirm oder ein Monitor.

In einer bevorzugten Ausführungsform weist das elektrische Gerät die Auswerteinheit auf. D.h., Das System weist das elektrische Gerät mit der Aufnahmeeinheit, der Ausgabeeinheit und der Auswerteinheit auf. In der Auswerteinheit sind die Standardflecken und die ihnen jeweils zugeordnete Behandlungsmethode hinterlegt. Vorzugsweise sind die Standardflecken thematisch d.h. entsprechend ihrer Behandlungsmethoden zusammengefasst. Die Standardflecken sind vorzugsweise für verschiedene Fleckfarben im ungewaschenen und gewaschenen Zustand eines Wäschestücks hinterlegt. Alternativ kann die Auswerteinheit auch eine Cloud, auch Rechnerwolke genannt, sein.

Bevorzugt ist das elektrische Gerät ausgebildet, von einem Nutzer des elektrischen Geräts Informationen abzufragen. Beispielsweise ist das elektrische Gerät ausgebildet, von dem Nutzer abzufragen, ob das Wäschestück mit dem aufgenommen Fleck bereits gewaschen wurde oder ungewaschen ist, und/oder um welche Textilart es sich bei dem Wäschestück handelt beispielsweise ob es sich um Weißwäsche, Buntwäsche usw. handelt. Dadurch kann eine Auswahl an für den aufgenommen Fleck zuordenbare Standardflecken eingegrenzt werden. Dadurch kann der aufgenommene Fleck einem oder mehreren Standardflecken weiterhin besser zugeordnet werden.

In einer bevorzugten Ausführungsform weist die Ausgabeeinheit eine grafische Benutzeroberfläche auf, welche ausgebildet ist, die Digitalaufnahme anzuzeigen und einen Nutzer auf der angezeigten Digitalaufnahme ein Segment der Digitalaufnahme als den Fleck kennzeichnen und einen weiteren Segment der Digitalaufnahme als fleckfrei kennzeichnen zu lassen. Dadurch kann eine korrekte Auswertung des aufgenommenen Flecks sichergestellt werden. So wird verhindert, dass bei einer automatisierten Erkennung einer Fleckenposition versehentlich beispielsweise ein Teil eines unverschmutzten Textilmusters als Fleck interpretiert wird. Die grafische Benutzeroberfläche, auch grafische Benutzerschnittstelle genannt, ist beispielsweise als Touchscreen ausgebildet.

Bevorzugter ist die grafische Benutzeroberfläche ausgebildet, dem Nutzer mehrere der hinterlegten Standardflecken anzuzeigen und anwählen zu lassen. Auf Basis der angewählten Standardflecken kann weiterhin die Behandlungsmethode eingegrenzter ermittelt und vorgeschlagen werden.

In einer bevorzugten Ausführungsform ist das elektrische Gerät in einer ersten Variante eine Waschmaschine. Die Waschmaschine weist bevorzugt die Aufnahmeeinheit, die Auswerteinheit und die Ausgabeneinheit auf. Eine Gerätsteuerung kann den Ablauf von der Aufnahme der Digitalaufnahme bis zur Ausgabe der Behandlungsmethode steuern. Die Waschmaschine ist vorzugsweise ausgelegt, Waschprogrammparameter für die Behandlungsmethode zu ermitteln. Waschprogrammparameter sind beispielsweise Waschtemperatur, Drehzahl einer Trommel der Waschmaschine, Waschzeit, zum Waschen eingesetzte Wassermenge, ob eine Vorwäsche und/oder ein Einweichen während es Programms durchgeführt wird oder nicht, Startzeitverzögerung etc. Diese Parameter können direkt in der Gerätesteuerung zur Verfügung stehen. Bei der Waschmaschine kann es sich um einen Waschautomaten oder ein Kombigerät wie ein Waschtrockner handeln.

Das elektrische Gerät ist in der ersten Variante bevorzugt ausgebildet, nach Durchführung der Behandlungsmethode mittels eines Waschprogramms einen Nutzer des elektrischen Geräts abzufragen, ob der Fleck mittels des Waschprogramms entfernt wurde, und in Abhängigkeit von einer Antwort des Nutzers auf die Abfrage ihm eine weitere Behandlungsmethode auszugeben. Wenn der Nutzer die Frage, ob der Fleck mittels des Waschprogramms entfernt wurde, bejaht, führt das elektrische Gerät in der ersten Variante keine weitere Aktion aus. Wenn der Nutzer die Frage, ob der Fleck mittels des Waschprogramms entfernt wurde, verneint, führt das elektrische Gerät in der ersten Variante eine weitere Aktion aus. Die Gerätsteuerung steuert einen weiteren Ablauf von der Aufnahme der Digitalaufnahme bis zur Ausgabe der Behandlungsmethode. D.h., der Fleck wird von der Waschmaschine erneut aufgenommen und ausgewertet, so dass anhand der weiteren Auswertung eine weitere Behandlungsmethode ausgegeben wird, die dann durchgeführt werden kann.

In einer anderen bevorzugten Ausführungsform ist das elektrische Gerät in einer zweiten Variante ein Mobiltelefon oder ein Computer wie beispielsweise ein Laptop oder ein Tablet. Besonders bevorzugt ist das elektrische Gerät in der zweiten Variante ein Smartphone. Beispielsweise weist das elektrische Gerät in der zweiten Variante eine App, auch Anwendungssoftware genannt, auf. Die App ist beispielsweise ausgebildet ist, die Aufnahmeeinheit zu starten, so dass sie eine Digitalaufnahme von dem Fleck auf dem Wäschestück aufnimmt, und/oder eine von der Aufnahmeeinheit aufgenommene und im elektrischen Gerät hinterlegte Digitalaufnahme anzuzeigen und vom Nutzer anwählen zu lassen. Ferner ist die App beispielsweise ausgebildet, mittels der Auswerteinheit des elektrischen Geräts anhand der Digitalaufnahme den Fleck auf dem Wäschestück derart auszuwerten, dass der Fleck mindestens einem Standardfleck aus mehreren in dem System hinterlegten Standardflecken zugeordnet wird, welchen jeweils eine Behandlungsmethode zum Entfernen des Flecks zugeordnet ist, oder an eine von dem elektrischen Gerät externe Auswerteinheit zu übermitteln, so dass diese die vorstehend beschriebene Auswertung des Flecks vornimmt. Ferner ist die App beispielsweise ausgebildet, mittels der Ausgabeeinheit des elektrischen Geräts die dem hinterlegten Fleck zugeordnete Behandlungsmethode zum Entfernen des Flecks automatisch auszugeben insbesondere anzuzeigen.

In der zweiten Variante ist das elektrische Gerät bevorzugt ausgebildet, Waschprogramm-Parameter für die ausgegebene Behandlungsmethode zu ermitteln.
Waschprogrammparameter sind beispielsweise Waschtemperatur, Drehzahl einer Trommel der Waschmaschine, Waschzeit, zum Waschen eingesetzte Wassermenge, ob eine Vorwäsche und/oder ein Einweichen während es Programms durchgeführt wird oder nicht, Startzeitverzögerung etc. Ferner ist das elektrische Gerät in der zweiten Variante bevorzugt ausgebildet, die Behandlungsmethode an eine Waschmaschine zu übermitteln. Bevorzugter ist das elektrische Gerät in der zweiten Variante ausgelegt, die ermittelten Waschprogrammparameter an die Waschmaschine zu übermitteln. Das elektrische Gerät ist vorzugsweise ausgelegt, die Übermittlung drahtlos auszuführen. Bei der Waschmaschine kann es sich um einen Waschautomaten oder ein Kombigerät wie ein Waschtrockner handeln. Bevorzugt sind sowohl das elektrischer Gerät in der zweiten Variante und die Waschmaschine ausgebildet bidirektional zu kommunizieren.

Das elektrische Gerät ist in der zweiten Variante bevorzugt ausgebildet, von der Waschmaschine nach Durchführung der Behandlungsmethode mittels eines Waschprogramms eine Mitteilung zu empfangen, nach Empfang der Mitteilung einen Nutzer des elektrischen Geräts abzufragen, ob der Fleck mittels der Behandlungsmethode entfernt wurde, und in Abhängigkeit von einer Antwort des Nutzers auf die Abfrage ihm eine weitere Behandlungsmethode auszugeben. Mittels der empfangenen Mitteilung wird das elektrische Gerät über die Durchführung der Behandlungsmethode informiert. Vorzugsweise ist das elektrische Gerät ausgebildet, nach Empfang der Mitteilung, automatisch eine Abfrage zu generieren, ob der Fleck mittels der Behandlungsmethode entfernt wurde. Das elektrische Gerät ist vorzugsweise ausgebildet, die Abfrage automatisch zu beenden, wenn der Nutzer diese Abfrage bejaht. Ferner ist das elektrische Gerät vorzugsweise ausgebildet, wenn der Nutzer die Abfrage, ob der Fleck mittels der Behandlungsmethode entfernt wurde, verneint, von dem Nutzer eine weitere Digitalaufnahme anzufordern beispielsweise durch Generieren einer Aufforderung, eine weitere Digitalaufnahme aufzunehmen, und/oder Starten der Aufnahmeeinheit, und durch eine integrale oder externe Aufnahmeeinheit einen in der weiteren Digitalaufnahme gezeigten Fleck auf einem Wäschestück auszuwerten und anschließend durch die Ausgabeeinheit eine weitere ermittelte Behandlungsmethode auszugeben, wie vorstehend bereits beschrieben.

Mittels des Systems kann ein Verfahren zur optischen Fleckenerkennung ausgeführt werden. Das Verfahren weist die folgenden Schritte auf: Aufnehmen einer Digitalaufnahme von zumindest einem Teil eines Wäschestücks, Auswerten eines Fleck auf dem Wäschestück anhand der Digitalaufnahme, so dass der Fleck mindestens einem Standardfleck aus mehreren in dem System hinterlegten Standardflecken zugeordnet wird, welchen jeweils eine Behandlungsmethode zum Entfernen des Flecks zugeordnet ist, und Ausgeben der dem hinterlegten Fleck zugeordneten Behandlungsmethode zum Entfernen des Flecks.

Ferner kann mittels des Systems ein Verfahren zur Behandlung eines Flecks auf einem Wäschestück ausgeführt werden. Das Verfahren weist die folgenden Schritte auf: Aufnehmen einer Digitalaufnahme von zumindest einem Teil eines Wäschestücks, Auswerten eines Fleck auf dem Wäschestück anhand der Digitalaufnahme, so dass der Fleck mindestens einem Standardfleck aus mehreren in dem System hinterlegten Standardflecken zugeordnet wird, welchen jeweils eine Behandlungsmethode zum Entfernen des Flecks zugeordnet ist, und Ausgeben der dem hinterlegten Fleck zugeordneten Behandlungsmethode zum Entfernen des Flecks und Durchführen eines Waschprogramms mit Waschprogrammparametern, die Waschprogrammparametern der ausgegeben Behandlungsmethode entsprechen. Bevorzugt wird der Nutzer nach der Durchführung des Waschprogramms abgefragt, ob der Fleck mittels des Waschprogramms entfernt wurde. In Abhängigkeit von einer Antwort des Nutzers werden die vorstehenden Schritte wiederholt. D.h., wenn der Nutzer "ja" antwortet, werden die Schritte nicht wiederholt. Wenn der Nutzer "nein" antwortet, werden die vorstehenden Schritte wiederholt, um den Fleck aus dem Wäschestück weiterhin entfernen zu können.

Ein Ausführungsbeispiel der Erfindung ist in den Zeichnungen rein schematisch dargestellt und wird nachfolgend näher beschrieben. Es zeigt schematisch und nicht maßstabsgerecht
- Fig. 1: ein System gemäß einer ersten Ausführungsform; und
- Fig. 2: ein System gemäß einer zweiten Ausführungsform.

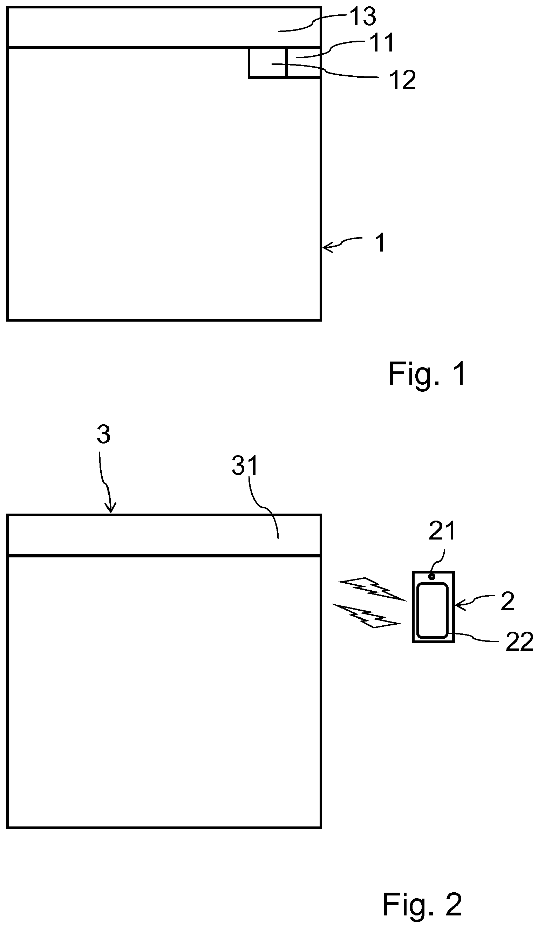

Fig. 1 zeigt ein System gemäß einer ersten Ausführungsform. Das System weist eine Waschmaschine als ein elektrisches Gerät 1 mit einer Aufnahmeeinheit 11 zum Aufnehmen einer Digitalaufnahme (nicht gezeigt) und einer Ausgabeeinheit 12 auf. Das elektrische Gerät 1 weist ferner eine Auswerteinheit (nicht gezeigt) zum Auswerten der Digitalaufnahme auf. Das elektrische Gerät weist ferner eine Blende 13 auf. Die Aufnahmeeinheit 11 ist eine Digitalkamera. Die Ausgabeeinheit 12 ist beispielsweise ein Display mit einer grafischen Benutzeroberfläche. Das System ist ausgebildet ist, mittels der Aufnahmeeinheit 11 eine Digitalaufnahme von zumindest einem Teil eines Wäschestücks aufzunehmen, mittels der Auswerteinheit anhand der Digitalaufnahme einen Fleck auf dem Wäschestück derart auszuwerten, dass der Fleck mindestens einem Standardfleck aus mehreren in dem System hinterlegten Standardflecken zugeordnet wird, welchen jeweils eine Behandlungsmethode zum Entfernen des Flecks zugeordnet ist, und mittels der Ausgabeeinheit 12 die dem hinterlegten Fleck zugeordnete Behandlungsmethode zum Entfernen des Flecks auszugeben.

Die Ausgabeeinheit 12 ist ausgebildet, die Digitalaufnahme anzuzeigen und einen Nutzer auf der angezeigten Digitalaufnahme ein Segment der Digitalaufnahme als den Fleck kennzeichnen zu lassen und ein weiteres Segment der Digitalaufnahme als fleckfrei kennzeichnen zu lassen. Ferner ist die Ausgabeeinheit 12 ausgebildet, dem Nutzer mehrere der hinterlegten Standardflecken anzuzeigen und anwählen zu lassen, wenn der Fleck mehreren Standardflecken zugeordnet wird.

Bei Betrieb kann ein Nutzer (nicht gezeigt) die Aufnahmeeinheit 11 über die Blende 13 oder die Ausgabeeinheit 12 oder die Aufnahmeeinheit 11 selbst starten, so dass das elektrische Gerät 1 eine Digitalaufnahme von zumindest einem Teil eines Wäschestücks (nicht gezeigt) mit Fleck (nicht gezeigt) aufnimmt. Die Digitalaufnahme wird von der Ausgabeeinheit 12 angezeigt. Auf der angezeigten Digitalaufnahme kann der Nutzer ein Segment der Digitalaufnahme als den Fleck kennzeichnen und ein weiteres Segment der Digitalaufnahme als fleckfrei kennzeichnen. Mittels der Auswerteinheit wird der Fleck anhand der Digitalaufnahme optisch ausgewertet, in dem die Fleckfarbe auf weißem Untergrund ermittelt wird. Über die Ausgabeeinheit 12 wird der Nutzer abgefragt, ob das Wäschestück bereits gewaschen wurde oder nicht und um welche Wäscheart wie Weißwäsche, Buntwäsche etc., es sich bei dem Wäschestück handelt. Die Auswerteinheit ordnet dann den Fleck mindestens einem Standardfleck aus mehreren in dem System hinterlegten Standardflecken zu, welchen jeweils eine Behandlungsmethode zum Entfernen des Flecks zugeordnet ist.

Dem Nutzer können mehrere der hinterlegten Standardflecken von der Ausgabeeinheit 12 angezeigt werden. Er kann einen oder mehrere der Standardflecken anwählen. Anhand all dieser Informationen werden mittels der Ausgabeeinheit 12 eine oder mehrere dem oder den angewählten hinterlegten Standardflecken zugeordnete Behandlungsmethoden zum Entfernen des Flecks ausgegeben. Ferner werden Waschprogrammparameter für die ausgegebene(n) Behandlungsmethode(n) ermittelt. Ferner wird dem Nutzer eine Aufforderung anzuzeigen, die oder eine der Behandlungsmethoden mittels Starten eines Waschprogramms mit den ermittelten Waschprogrammparametern zu starten. Wenn der Nutzer die Behandlungsmethode startet, wird die ausgegebene Behandlungsmethode von dem elektrischen Gerät 1 durchgeführt. Nach Durchführung der Behandlungsmethode mittels des Waschprogramms wird der Nutzer des elektrischen Geräts 1 weiterhin abgefragt, ob der Fleck mittels der Behandlungsmethode entfernt wurde. Wenn nein, wird auf der Ausgabeeinheit 12 eine Aufforderung angezeigt, eine weitere Digitalaufnahme aufzunehmen und das vorstehende Verfahren wird wiederholt. Wenn ja, wird keine weitere Aktion durchgeführt.

Fig. 2 zeigt ein System gemäß einer zweiten Ausführungsform. Das System weist ein Smartphone als ein elektrisches Gerät 2 mit einer Aufnahmeeinheit 22 zum Aufnehmen einer Digitalaufnahme (nicht gezeigt) und einer Ausgabeeinheit 22 auf. Das elektrische Gerät 2 weist ferner eine Auswerteinheit (nicht gezeigt) zum Auswerten der Digitalaufnahme auf. Die Aufnahmeeinheit 22 ist eine Digitalkamera. Die Ausgabeeinheit 22 ist eine Anzeige insbesondere mit einer grafischen Benutzeroberfläche. Das System ist ausgebildet ist, mittels der Aufnahmeeinheit 22 eine Digitalaufnahme von zumindest einem Teil eines Wäschestücks aufzunehmen, mittels der Auswerteinheit anhand der Digitalaufnahme einen Fleck auf dem Wäschestück derart auszuwerten, dass der Fleck mindestens einem Standardfleck aus mehreren in dem System hinterlegten Standardflecken zugeordnet wird, welchen jeweils eine Behandlungsmethode zum Entfernen des Flecks zugeordnet ist, und mittels der Ausgabeeinheit 22 die dem hinterlegten Fleck zugeordnete Behandlungsmethode zum Entfernen des Flecks auszugeben.

Die Ausgabeeinheit 12 ist ausgebildet, die Digitalaufnahme anzuzeigen und einen Nutzer auf der angezeigten Digitalaufnahme ein Segment der Digitalaufnahme als den Fleck und ein weiteres Segment der Digitalaufnahme als fleckfrei kennzeichnen zu lassen. Ferner ist die Ausgabeeinheit 12 ausgebildet, dem Nutzer mehrere der hinterlegten Standardflecken anzuzeigen und anwählen zu lassen. Das elektrische Gerät 2 ist ferner ausgebildet mit der Waschmaschine 3, die eine Blende 31 aufweist, über die ein Waschprogramm startbar ist, bidirektional und drahtlos zu kommunizieren.

Bei Betrieb läuft das zu Fig. 1 beschriebene Verfahren ab mit dem Unterschied, dass das elektrische Gerät 2 die ermittelten Waschprogrammparameter an die Waschmaschine 3 übermittelt, so dass der Nutzer über die Blende 31 ein der Behandlungsmethode entsprechendes Waschprogramm mit den ermittelten Waschprogrammparametern starten kann, damit das Wäschestück in der Waschmaschine 3 gewaschen wird, und dass das elektrische Gerät 2 nach Durchführung der Behandlungsmethode mittels des Waschprogramms eine Mitteilung von der Waschmaschine 3 empfängt, nach Empfang der Mitteilung den Nutzer des elektrischen Geräts 2 abfragt, ob der Fleck mittels der Behandlungsmethode entfernt wurde, und in Abhängigkeit von der Antwort des Nutzers auf die Abfrage ihm eine weitere Behandlungsmethode ausgibt.

### Bezugszeichenliste

- 1: elektrisches Gerät
- 11: Aufnahmeeinheit
- 12: Ausgabeeinheit
- 13: Blende
- 2: elektrisches Gerät
- 21: Aufnahmeeinheit
- 22: Ausgabeeinheit
- 3: Waschmaschine
- 31: Blende

## Patentansprüche

1. System, welches
- ein elektrisches Gerät (1, 2) mit einer Aufnahmeeinheit (11, 21) zum Aufnehmen einer Digitalaufnahme und einer Ausgabeeinheit (12, 22) und
- eine Auswerteinheit zum Auswerten der Digitalaufnahme aufweist und
- welches ausgebildet ist,
▪ mittels der Aufnahmeeinheit (11, 21) eine Digitalaufnahme von zumindest einem Teil eines Wäschestücks aufzunehmen,
▪ mittels der Auswerteinheit anhand der Digitalaufnahme einen Fleck auf dem Wäschestück derart auszuwerten, dass der Fleck mindestens einem Standardfleck aus mehreren in dem System hinterlegten Standardflecken zugeordnet wird, welchen jeweils eine Behandlungsmethode zum Entfernen des Flecks zugeordnet ist, und
▪ mittels der Ausgabeeinheit (12, 22) die dem hinterlegten Fleck zugeordnete Behandlungsmethode zum Entfernen des Flecks auszugeben.

2. System nach Anspruch 1, **dadurch gekennzeichnet, dass** das elektrische Gerät (1, 2) die Auswerteinheit aufweist.

3. System nach Anspruch 1 oder 2, **dadurch gekennzeichnet, dass** das elektrische Gerät (1, 2) ausgebildet ist, von einem Nutzer des elektrischen Geräts (1, 2) Informationen abzufragen.

4. System nach einem der vorangehenden Ansprüche, **dadurch gekennzeichnet, dass** die Ausgabeeinheit (12, 22) eine grafische Benutzeroberfläche (12, 22) aufweist, welche ausgebildet ist, die Digitalaufnahme anzuzeigen und einen Nutzer auf der angezeigten Digitalaufnahme ein Segment der Digitalaufnahme als den Fleck kennzeichnen und ein weiteres Segment der Digitalaufnahme als fleckfrei kennzeichnen zu lassen.

5. System nach Anspruch 4, **dadurch gekennzeichnet, dass** die grafische Benutzeroberfläche (12, 22) ausgebildet ist, dem Nutzer mehrere der hinterlegten Standardflecken anzuzeigen und anwählen zu lassen.

6. System nach einem der vorangehenden Ansprüche, **dadurch gekennzeichnet, dass** das elektrische Gerät (1) eine Waschmaschine ist.

7. System nach Anspruch 6, **dadurch gekennzeichnet, dass** das elektrische Gerät (1) ausgebildet ist, nach Durchführung der Behandlungsmethode mittels eines Waschprogramms einen Nutzer des elektrischen Geräts (1) abzufragen, ob der Fleck mittels des Waschprogramms entfernt wurde, und in Abhängigkeit von einer Antwort des Nutzers auf die Abfrage ihm eine weitere Behandlungsmethode auszugeben.

8. System nach einem der vorangehenden Ansprüche 1 bis 5, **dadurch gekennzeichnet, dass** das elektrische Gerät (2) ein Mobiltelefon oder ein Computer ist.

9. System nach Anspruch 8, **dadurch gekennzeichnet, dass** das elektrische Gerät (2) ausgebildet ist, Waschprogramm-Parameter für die ausgegebene Behandlungsmethode zu ermitteln.

10. System nach Anspruch 8 oder9, **dadurch gekennzeichnet, dass** das elektrische Gerät (2) ausgebildet ist, die Behandlungsmethode an eine Waschmaschine (3) zu übermitteln.

11. System nach Anspruch 10, **dadurch gekennzeichnet, dass** das elektrische Gerät (2) ausgebildet ist, von der Waschmaschine (3) nach Durchführung der Behandlungsmethode mittels eines Waschprogramms eine Mitteilung zu empfangen, nach Empfang der Mitteilung einen Nutzer des elektrischen Geräts (2) abzufragen, ob der Fleck mittels der Behandlungsmethode entfernt wurde, und in Abhängigkeit von einer Antwort des Nutzers auf die Abfrage ihm eine weitere Behandlungsmethode auszugeben.
